# EUROPÄISCHE PATENTANMELDUNG

(11) **EP 3 895 644 A1**
(43) Veröffentlichungstag der Anmeldung: **20.10.2021**
(21) Anmeldenummer: 20169631.7
(22) Anmeldetag: 15.04.2020
(51) Int. Cl.: A61B 18/14, A61B 17/28, A61B 17/285

(54) **CHIRURGISCHES INSTRUMENT**

(71) Anmelder: Erbe Elektromedizin GmbH, 72072 Tübingen (DE)
(72) Erfinder: BOB, felix, 72108 Rottenburg (DE)
(74) Vertreter: Rüger Abel Patentanwälte PartGmbB

(57) **Zusammenfassung**

Die Erfindung betrifft eine Betätigungsvorrichtung (30) zur Betätigung eines Werkzeugs (24) eines chirurgischen Instruments (20). Die Betätigungsvorrichtung (30) hat ein Betätigungselement (31), das zwischen einer Ausgangslage (I) und einer Arbeitslage (W) schwenkbar gelagert ist. Mittels einer Kopplungseinrichtung (35), die ein Kopplungsglied (36) aufweist oder durch das Kopplungsglied (36) gebildet ist, ist das Betätigungselement (31) mit dem Werkzeug (24) bewegungsgekoppelt. Das Kopplungsglied (36) ist elastisch verformbar und bewirkt bei einer Auslenkung des Betätigungselements (31) aus der Ausgangslage (I) durch die elastische Verformung ein Rückstellmoment (MR), das das Betätigungselement (31) zurück in die Ausgangslage (I) drängt. Zusätzliche federelastische Rückstellelemente können dadurch entfallen.

## Beschreibung

Die Erfindung betrifft ein chirurgisches Instrument, beispielsweise ein elektrochirurgisches Instrument. Das chirurgische Instrument kann zwei schwenkbar aneinander gelagerte Branchen aufweisen. An dem chirurgischen Instrument ist ein in einer Längsrichtung geführt bewegbar gelagertes Werkzeug vorhanden, z.B. ein Messer. Das Messer kann in einem Messerkanal gelagert sein. Über ein Betätigungselement kann das Messer in Längsrichtung bewegt werden, beispielsweise um zwischen den Branchen eingeklemmtes biologisches Gewebe zu durchtrennen.

EP 2 436 327 A1 beschreibt eine chirurgische Klemme mit zwei schwenkbar aneinander gelagerten Branchen und einem in einer Längsrichtung geführten Messer. Über ein schwenkbar gelagertes Betätigungselement kann das Messer in Längsrichtung bewegt werden. Um das Betätigungselement in eine Ausgangsstellung vorzuspannen, ist eine Schraubenfeder vorhanden, die beim Auslenken des Betätigungselements aus seiner Ausgangslage auf Zug beansprucht wird und ein Rückstellmoment in Richtung der Ausgangslage erzeugt.

US 2017/0209205 A1 beschreibt eine bipolare chirurgische Klemme mit zwei schwenkbar aneinander gelagerten Branchen und einem in einer Längsrichtung geführt gelagerten Messer. Über ein Betätigungselement kann das Messer in Längsrichtung bewegt werden. Das Betätigungselement ist über eine Kopplungseinrichtung mit dem Messer bewegungsgekoppelt, wobei die Kopplungseinrichtung einen Teleskophebel aufweist, um beim Schwenken des Betätigungselements um eine Schwenkachse den benötigten Raum für die Schwenkbewegung gering halten zu können. Beim Schwenken des Betätigungselements von einer Ausgangslage in eine Arbeitslage wird der Teleskophebel beim Kontakt mit einer Gehäusewand zunächst eingefahren und anschließend wieder ausgefahren. Dadurch soll das Gehäuse klein gehalten werden können. Zusätzlich ist ein nicht dargestelltes Vorspannelement vorhanden, das einerseits am Teleskophebel und andererseits am Betätigungselement angreift, um das Betätigungselement in seiner Ausgangsstellung vorzuspannen.

Die bislang bekannten Anordnungen sind konstruktiv aufwendig, was insbesondere bei Einweginstrumenten zu hohen Kosten führt. Bei Mehrweginstrumenten ist das Reinigen und Sterilisieren problematisch, insbesondere wenn Schraubenfedern oder teleskopierbare Anordnungen eingesetzt werden. Dort können Verschmutzungen nicht oder nur mit großem Aufwand entfernt werden.

Es kann daher als Aufgabe der vorliegenden Erfindung angesehen werden, ein chirurgisches Instrument zu schaffen, das einen kostengünstigen Aufbau und eine einfache Reinigung gewährleistet.

Diese Aufgabe wird durch ein chirurgisches Instrument mit den Merkmalen des Patentanspruches 1 gelöst.

Das erfindungsgemäße chirurgische Instrument weist ein Werkzeug auf, beispielsweise ein Messer, das in einer Längsrichtung bewegbar angeordnet ist. Zur Betätigung des Werkzeugs ist ein Betätigungselement vorhanden. Das Betätigungselement ist schwenkbar gelagert. Es kann zwischen einer Ausgangslage und einer Arbeitslage geschwenkt werden.

Über eine Kopplungseinrichtung wird eine Bewegungskopplung zwischen dem Betätigungselement und dem Werkzeug hergestellt. Die Kopplungseinrichtung weist ein Kopplungsglied auf. Das Kopplungsglied ist dazu eingerichtet, die Schwenkbewegung des Betätigungselements auf das Werkzeug und/oder ein mit dem Werkzeug insbesondere unbeweglich verbundenes Teil zu übertragen. Das Kopplungsglied bildet bei einer bevorzugten Ausführungsform ein Pleuel der Kopplungseinrichtung. Das vorzugsweise vom Kopplungsglied gebildete Pleuel ist an einem Ende gelagert, um eine Drehbewegung oder Schwenkbewegung auszuführen und am anderen Ende gelagert, um eine im Wesentlichen geradlinige Bewegung in Längsrichtung auszuführen.

Das Kopplungsglied ist elastisch verformbar. Vorzugsweise ist das Kopplungsglied in der Ausgangslage des Betätigungselements elastisch nicht verformt oder elastisch weniger stark verformt als in der Arbeitslage des Betätigungselements und nimmt einen ersten Zustand ein. In der Arbeitslage des Betätigungselements ist das Kopplungsglied elastisch stärker verformt als in seinem ersten Zustand und bewirkt ein Rückstellmoment auf das schwenkbar gelagerte Betätigungselement in Richtung der Ausgangslage. Der Zustand des elastisch verformten Kopplungsglied in der Arbeitslage des Betätigungselements wird als zweiter Zustand bezeichnet.

Erfindungsgemäß bewirkt somit das Kopplungsglied selbst das Rückstellmoment auf das Betätigungselement, um dieses in die Ausgangslage zurückzubewegen. Auf zusätzliche Elemente zur Erzeugung des Rückstellmoments und insbesondere auf Schraubenfedern kann verzichtet werden.

Es ist vorteilhaft, wenn die Kopplungseinrichtung und beispielsweise das Kopplungsglied lösbar mit dem Werkzeug verbunden ist. Diese lösbare Verbindung kann insbesondere kraftschlüssig und/oder formschlüssig sein und beispielsweise durch eine Rastverbindung realisiert werden. Dadurch kann das Werkzeug ausgetauscht werden und die Kopplungseinrichtung bzw. das Kopplungsglied kann im Instrument verbleiben. Alternativ oder zusätzlich kann eine solche lösbare Verbindung auch an einer anderen Stelle der Kopplungseinrichtung vorhanden sein, beispielsweise an der Verbindung zwischen der Kopplungseinrichtung und dem Betätigungselement.

Die lösbare Verbindung kann bei einem Ausführungsbeispiel durch eine Nut im Werkzeug realisiert werden, in die die Kopplungseinrichtung bzw. das Kopplungsglied eingreift. Durch eine elastische Verformungskraft im ersten Zustand des Kopplungsgliedes wird zumindest eine kraftschlüssige Verbindung bewirkt.

Vorzugsweise nimmt das Werkzeug in der Ausgangslage des Betätigungselements eine eingefahrene Stellung ein. In der Arbeitslage des Betätigungselements kann das Werkzeug eine ausgefahrene Stellung einnehmen. In einer eingefahrenen Stellung ist das Werkzeug ohne Einfluss auf biologisches Gewebe. In einer ausgefahrenen Stellung kann das Werkzeug auf biologisches Gewebe einwirken.

Das chirurgische Instrument kann vorzugsweise zwei schwenkbar aneinander gelagerte Branchen aufweisen. Das Werkzeug ist in einer der Branchen in Längsrichtung bewegbar gelagert, beispielsweise in einem Werkzeugkanal.

Das Werkzeug kann ein Messer sein, wobei der Werkzeugkanal einen Messerkanal bildet. Beispielsweise kann ein Messer in der ausgefahrenen Stellung biologisches Gewebe schneiden.

Bevorzugt verbindet das Kopplungsglied, beispielsweise das Pleuel, zwei mit Abstand zueinander angeordnete Teile. Diese Teile können vom Betätigungselement oder einem damit bewegungsgekoppelten Teil einerseits und vom Werkzeug oder einem damit bewegungsgekoppelten Verbindungsteil andererseits gebildet werden. Ohne das Kopplungsglied wäre die Bewegungsübertragung vom Betätigungselement auf das Werkzeug nicht möglich.

Das Kopplungsglied ist insbesondere integral einstückig ausgebildet. Es ist aus einem elastisch verformbaren Material hergestellt, beispielsweise aus einem metallischen Material, insbesondere einer metallischen Legierung. Optional kann es mit einer Schutzschicht versehen sein, um das Kopplungsglied gegen chemische Einwirkungen zu schützen, wobei die Schutzschicht für die Bewegungskopplung keine Bedeutung hat.

Bei einer bevorzugten Ausführungsform ist das Kopplungsglied mit einem Ende unmittelbar am Betätigungselement mit Abstand zur Schwenkachse des Betätigungselements angebracht. Mit dem jeweils anderen Ende kann das Kopplungsglied unmittelbar mit dem Werkzeug oder einem mit dem bewegungsgekoppelten Verbindungsteil angeordnet sein. Insbesondere ist das Verbindungsteil unbeweglich am Werkzeug angeordnet. Das Kopplungsglied kann dazu eingerichtet sein, eine Schwenkbewegung des Betätigungselements in eine lineare Bewegung des Werkzeugs zu übersetzen. Das Kopplungsglied kann das einzige Teil sein, das für die Übersetzung der Schwenkbewegung in die lineare Bewegung vorhanden ist.

Es ist vorteilhaft, wenn das Kopplungsglied eine bügelförmige Gestalt hat. Es kann beispielsweise als Drahtbügel oder Stanzbügel ausgebildet sein. Insbesondere kann das Kopplungsglied wenigstens einen Längsschenkel und einen sich daran anschließenden Querschenkel aufweisen. Der Querschenkel erstreckt sich insbesondere rechtwinklig zur Bewegungsrichtung des Werkzeugs, also rechtwinklig zur Längsrichtung. Der wenigstens eine Längsschenkel erstreckt sich vorzugsweise unter einem Winkel geneigt zur Längsrichtung, der einen Betrag zwischen 0 Grad und 90 Grad aufweist, vorzugsweise zwischen 10 Grad und 80 Grad und weiter vorzugsweise zwischen 20 Grad und 70 Grad.

Es ist insbesondere vorteilhaft, wenn das Kopplungsglied zwei und insbesondere genau zwei Längsschenkel aufweist, die über den Querschenkel miteinander verbunden sind. Der Querschenkel bildet vorzugsweise ein Ende des Kopplungsgliedes und insbesondere das Ende, das dem Werkzeug zugeordnet ist.

Jeder Längsschenkel kann ein freies Schenkelende aufweisen, die vorzugsweise nicht unmittelbar miteinander verbunden sind und weiter vorzugsweise nicht unmittelbar aneinander anliegen. Insbesondere sind die freien Schenkelenden der Längsschenkel in einer Ebene, die rechtwinklig zur Querrichtung ausgerichtet ist, versetzt zueinander angeordnet. Insbesondere sind die freien Schenkelenden innerhalb dieser Ebene entlang einer gemeinsamen Geraden in Erstreckungsrichtung der Längsschenkel angeordnet, wenn sich das Kopplungsglied in einem unverformten Ruhezustand befindet, der beispielsweise dem ersten Zustand entsprechen kann.

Die Längsschenkel des Kopplungsgliedes können unterschiedlich lang sein.

Es ist bevorzugt, wenn sich die Längsschenkel und der Querschenkel in der Ausgangslage des Betätigungselements in einer gemeinsamen Ebene erstrecken. Außerhalb der Ausgangslage und insbesondere in der Arbeitslage des Betätigungselements können die Längsschenkel einen Winkel größer als Null einschließen. Der eingeschlossene Winkel zwischen den Längsschenkeln ist in der Arbeitslage am größten und nimmt zur Ausgangslage hin ab, wobei er in der Ausgangslage vorzugsweise gleich Null ist.

Es ist insbesondere vorteilhaft, wenn die Längsschenkel im zweiten Zustand des Kopplungsgliedes (Arbeitslage des Betätigungselements) elastisch unverformt sind verglichen mit dem ersten Zustand des Kopplungsgliedes (Ausgangslage des Betätigungselements). Wenn sich die Längsschenkel im ersten Zustand des Kopplungsgliedes beispielsweise geradlinig erstrecken, erstrecken sie sich im zweiten Zustand des Kopplungsgliedes bevorzugt ebenfalls im Wesentlichen geradlinig. Die Längsschenkel können also insbesondere biegesteif gegenüber einer Biegung quer zu ihrer Erstreckungsrichtung sein. Bei dieser Ausgestaltung kann eine in Längsrichtung auf das Werkzeug von außen einwirkende Kraft sehr gut über das Kopplungsglied am Betätigungselement abgestützt werden. Das Werkzeug wird somit nicht unerwünscht aus seiner ausgefahrenen Stellung in die eingefahrene Stellung zurückgedrängt. Dieser Abstützeffekt ist insbesondere dann verbessert, wenn die Längsschenkel in der Arbeitslage einen Winkel größer als Null einschließen. Der Winkel zwischen den Längsschenkeln wird in einer Projektionsebene gemessen, die rechtwinklig zur Querrichtung und somit rechtwinklig zum Querschenkel ausgerichtet ist.

Sollten sich die Längsschenkel im ersten Zustand des Kopplungsgliedes gekrümmt und/oder abgewinkelt und/oder abgeknickt erstrecken, kann diese Form im zweiten Zustand des Kopplungsgliedes aufrechterhalten bleiben.

Unabhängig von der Anzahl der Längsschenkel ist der Querschenkel im zweiten Zustand des Kopplungsgliedes (Arbeitslage des Betätigungselements) vorzugsweise um seine Erstreckungsrichtung tordiert. Diese Torsion erzeugt ein Drehmoment auf den wenigstens einen mit dem Querschenkel verbundenen Längsschenkel, wodurch das Rückstellmoment bewirkt wird.

Es ist vorteilhaft, wenn sich das Kopplungsglied im ersten Zustand geradlinig oder weniger stark gekrümmt zwischen seinen beiden Enden erstreckt als im zweiten Zustand. Zumindest im zweiten Zustand kann sich das Kopplungsglied gekrümmt zwischen seinen beiden Enden erstrecken. Bei dieser Ausführungsform kann das Kopplungsglied eine bügelförmige Gestalt aufweisen oder nach Art einer Blattfeder als flächiges Element ausgebildet sein. Das Kopplungsglied kann bei dieser Ausführungsform an einem Ende oder an beiden Enden drehfest befestigt sein, insbesondere an einem Ende drehfest mit dem Betätigungselement und/oder am anderen Ende drehfest mit dem Werkzeug oder einem mit dem Werkzeug insbesondere unbeweglich verbundenen Verbindungsteil.

Bei einem Ausführungsbeispiel kann das zumindest außerhalb seines ersten Zustandes elastisch verformte Kopplungsglied eine Kraft oder Kraftkomponente rechtwinklig zur Längsrichtung und insbesondere auch rechtwinklig zur Querrichtung auf das Werkzeug oder ein mit dem Werkzeug verbundenes Verbindungsteil ausüben. Auf diese Weise ist es beispielsweise möglich, das Werkzeug oder das Verbindungsteil in wenigstens einer vorgegebenen Position in Längsrichtung zu einzurasten.

Vorteilhafte Ausgestaltungen des erfindungsgemäßen chirurgischen Instruments ergeben sich aus den abhängigen Patentansprüchen, der Beschreibung und den Zeichnungen. Nachfolgend werden bevorzugte Ausführungsbeispiele anhand der beigefügten Zeichnungen im Einzelnen erläutert. Die Figuren der Zeichnungen zeigen:
Figuren 1 und 2 jeweils eine schematische Seitenansicht eines chirurgischen Instruments, insbesondere eines elektrochirurgischen Instruments,
Figur 3 ein Ausführungsbeispiel einer Betätigungsvorrichtung des chirurgischen Instruments in einer schematischen Seitenansicht, wobei sich ein Betätigungselement der Betätigungsvorrichtung in einer Ausgangslage befindet und ein Kopplungsglied einer Kopplungseinrichtung einen ersten Zustand einnimmt,
Figur 4 die Betätigungsvorrichtung aus Figur 3 in einer schematischen Seitenansicht, wobei sich das Betätigungselement in einer Arbeitslage befindet und ein Kopplungsglied einer Kopplungseinrichtung einen zweiten Zustand einnimmt,
Figur 5 ein Ausführungsbeispiel eines Kopplungsgliedes der Betätigungsvorrichtung aus den Figuren 3 und 4 in einer Draufsicht,
Figur 6 ein weiteres Ausführungsbeispiel einer Betätigungsvorrichtung für das chirurgische Instrument in einer schematischen Seitenansicht, wobei sich ein Betätigungselement der Betätigungsvorrichtung in einer Ausgangslage befindet und ein Kopplungsglied einer Kopplungseinrichtung einen ersten Zustand einnimmt,
Figur 7 das Ausführungsbeispiel der Betätigungsvorrichtung aus Figur 6 in einer schematischen Seitenansicht, wobei sich das Betätigungselement in einer Arbeitslage befindet und ein Kopplungsglied einer Kopplungseinrichtung einen zweiten Zustand einnimmt,
Figur 8 eine perspektivische Darstellung eines Ausführungsbeispiels eines Kopplungsgliedes der Betätigungsvorrichtung gemäß der Figuren 6 und 7,
Figur 9 ein weiteres Ausführungsbeispiel einer Betätigungsvorrichtung für das chirurgische Instrument in einer schematischen Seitenansicht, wobei sich ein Betätigungselement der Betätigungsvorrichtung in einer Ausgangslage befindet und ein Kopplungsglied einer Kopplungseinrichtung einen ersten Zustand einnimmt,
Figur 10 das Betätigungselement, ein Kopplungsglied und ein Verbindungsteil der Betätigungsvorrichtung aus Figur 9 in schematischer Einzeldarstellung,
Figur 11 das Kopplungsglied der Betätigungsvorrichtung gemäß der Figuren 9 und 10 in einer schematischen Seitenansicht, wobei das Kopplungsglied elastisch verformt ist und seinen zweiten Zustand einnimmt,
Figur 12 ein weiteres Ausführungsbeispiel einer Betätigungsvorrichtung für das chirurgische Instrument in einer schematischen Seitenansicht, wobei sich ein Betätigungselement der Betätigungsvorrichtung in einer Ausgangslage befindet und ein Kopplungsglied einer Kopplungseinrichtung einen ersten Zustand einnimmt,
Figur 13 das Ausführungsbeispiel der Betätigungsvorrichtung aus Figur 12 in einer schematischen Seitenansicht, wobei sich das Betätigungselement in einer Arbeitslage befindet und ein Kopplungsglied einer Kopplungseinrichtung einen zweiten Zustand einnimmt,
Figuren 14 und 15 jeweils eine schematische Prinzipdarstellung eines Kopplungsgliedes der Betätigungsvorrichtung gemäß der Figuren 12 und 13 in einer Draufsicht,
Figur 16 ein weiteres Ausführungsbeispiel einer Betätigungsvorrichtung für das chirurgische Instrument in einer schematischen Seitenansicht, wobei sich ein Betätigungselement der Betätigungsvorrichtung in einer Ausgangslage befindet und ein Kopplungsglied einer Kopplungseinrichtung einen ersten Zustand einnimmt und
Figur 17 das Ausführungsbeispiel der Betätigungsvorrichtung aus Figur 16 in einer schematischen Seitenansicht, wobei sich das Betätigungselement in einer Arbeitslage befindet und ein Kopplungsglied einer Kopplungseinrichtung einen zweiten Zustand einnimmt.

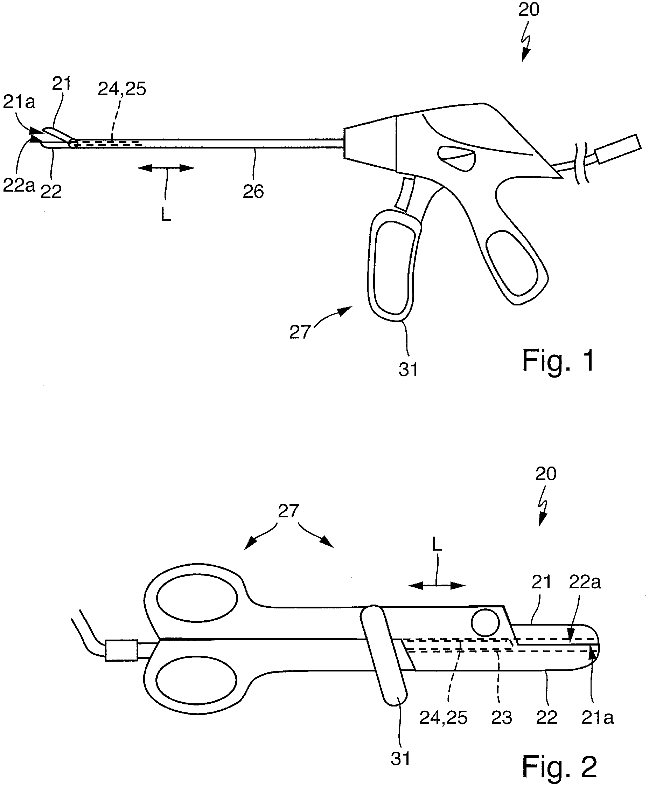

In den Figuren 1 und 2 sind schematisiert unterschiedliche Ausführungsbeispiele eines chirurgischen Instruments 20 veranschaulicht. Das chirurgische Instrument 20 kann, wie dargestellt, als elektrochirurgisches Instrument ausgebildet sein. Es kann sich um ein monopolares oder ein bipolares elektrochirurgisches Instrument handeln. Das chirurgische Instrument ist insbesondere als Mehrweginstrument ausgebildet. Es muss daher nach jeder Benutzung gereinigt und sterilisiert werden.

Das chirurgische Instrument 20 hat bei den veranschaulichten Ausführungsbeispielen zwei schwenkbar aneinander gelagerte Branchen 21, 22. Jede Branche 21, 22 hat eine Gewebekontaktfläche 21a bzw. 22a. Die Branchen 21, 22 können aufeinander zu oder voneinander weg bewegt werden, um ein biologisches Gewebe zwischen den Gewebekontaktflächen 21a, 22a einzuklemmen oder loszulassen.

Das chirurgische Instrument 20 weist außerdem ein Werkzeug 24 auf. Das Werkzeug 24 ist in einer Längsrichtung L bewegbar in einem Werkzeugkanal 23 gelagert. Das Werkzeug 24 kann beispielsweise durch ein Messer 25 gebildet sein. Der Werkzeugkanal 23 kann sich innerhalb eines sich an die Branchen 21, 22 anschließenden Schaftes 26 erstrecken (Figur 1) und/oder innerhalb einer der Branchen 21, 22 verlaufen (Figur 2).

Das Instrument 20 weist eine Bedieneinheit 27 auf. Mittels der Bedieneinheit 27 können die Branchen 21, 22 gegeneinander verschwenkt und das Werkzeug 24 in Längsrichtung L bewegt werden. Bei elektrochirurgischen Instrumenten kann mittels der Bedieneinheit 27 außerdem eine elektrische Spannung an die Gewebekontaktflächen 21a, 22a angelegt werden.

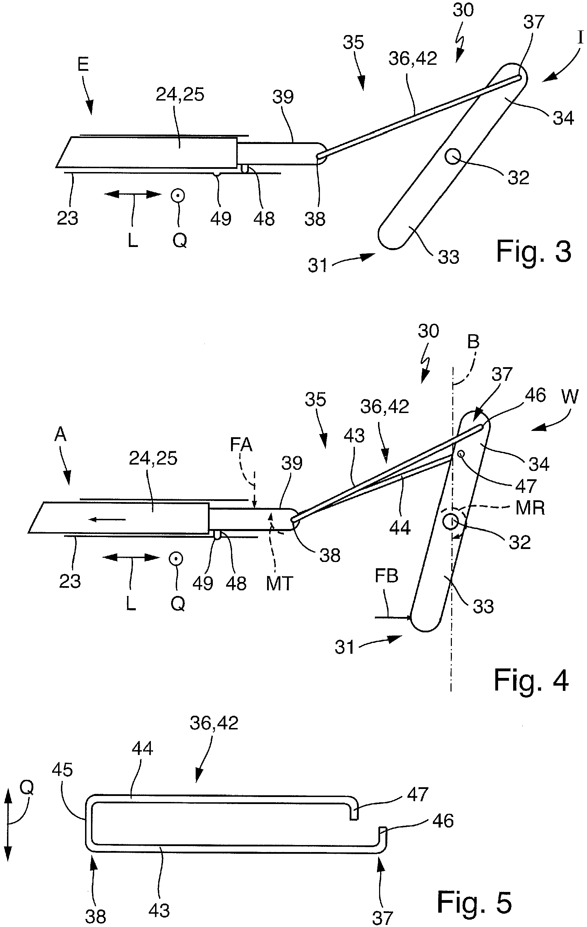

Das Werkzeug 24 und beispielsgemäß das Messer 25 kann zwischen einer eingefahrenen Stellung E und einer ausgefahrenen Stellung A in Längsrichtung L bewegt werden. Dazu weist das Instrument 20 eine Betätigungsvorrichtung 30 auf. In den Figuren 3-17 sind unterschiedliche Ausführungsformen der Betätigungsvorrichtung 30 oder Bestandteilen davon schematisch veranschaulicht.

Die Betätigungsvorrichtung 30 weist ein Betätigungselement 31 auf, das Bestandteil der Bedieneinheit 27 zur Bedienung des Instruments 20 ist. Das Betätigungselement 31 kann als separates Bedienelement der Bedieneinheit 27 ausgebildet sein (Figur 2) oder alternativ durch ein Bedienelement gebildet sein, das auch eine oder mehrere weitere Funktionen aufweist (Figur 1).

Das Betätigungselement 31 ist schwenkbar um eine Schwenkachse gelagert. Die Schwenkachse erstreckt sich beim Ausführungsbeispiel in einer Querrichtung Q, die rechtwinklig zur Längsrichtung L ausgerichtet ist. Die Schwenkachse ist durch ein Schwenkgelenk 32 definiert, mittels dem das Betätigungselement 31 schwenkbar an einem ortsfest bzw. unbeweglich gegenüber dem Gehäuse des Instruments 20 angeordneten Teil gelagert ist. Das Betätigungselement 31 hat zwei sich ausgehend vom Schwenkgelenk 32 erstreckende Arme 33, 34. Die Arme 33, 34 können sich in unterschiedliche und insbesondere entgegengesetzte Richtungen erstrecken, wie es beim Ausführungsbeispiel gezeigt ist.

Ein erster Arm 33 dient zur manuellen Betätigung. Ein zweiter Arm 34 ist über eine Kopplungseinrichtung 35 mit dem Werkzeug 24 bewegungsgekoppelt.

Die Kopplungseinrichtung 35 weist ein elastisch verformbares Kopplungsglied 36 auf. Beim Ausführungsbeispiel ist die Kopplungseinrichtung 35 durch das Kopplungsglied 36 gebildet. Zusätzliche bewegungsübertragende Bauteile können entfallen. Das Kopplungsglied 36 ist beispielsgemäß mit einem ersten Ende 37 am Betätigungselement 31 und insbesondere dem zweiten Arm 34 angeordnet. Vom ersten Ende 37 erstreckt sich das Kopplungsglied 36 zu einem zweiten Ende 38, das dem Werkzeug 24 oder einem mit dem Werkzeug 24 vorzugsweise unbeweglich verbundenen Verbindungsteil 39 zugeordnet ist. Bei den hier veranschaulichten Ausführungsbeispielen ist das zweite Ende 38 am Verbindungsteil 39 angeordnet. In Abwandlung hierzu könnte es auch unmittelbar am Werkzeug 24 angeordnet sein.

Durch die Bewegungskopplung zwischen dem Werkzeug 24 und dem Betätigungselement 31 mittels der Kopplungseinrichtung 35 kann das Schwenken des Betätigungselements 31 um die Schwenkachse bzw. das Schwenkgelenk 32 in eine Bewegung des Werkzeugs 24 in Längsrichtung L übersetzt werden, insbesondere um das Werkzeug 24 zwischen der eingefahrenen Stellung E und der ausgefahrenen Stellung A zu bewegen. Wenn sich das Werkzeug 24 in der eingefahrenen Stellung E befindet, befindet sich das Betätigungselement 31 in einer Ausgangslage I (Figuren 3, 6, 9, 12, 16). Wenn das Werkzeug 24 die ausgefahrene Stellung A einnimmt, befindet sich das Betätigungselement 31 in einer Arbeitslage W (Figuren 4, 7, 13, 17).

Um das Betätigungselement 31 aus der Ausgangslage I in die Arbeitslage W zu schwenken, kann eine Bedienperson auf den ersten Arm 33 eine Betätigungskraft FB ausüben.

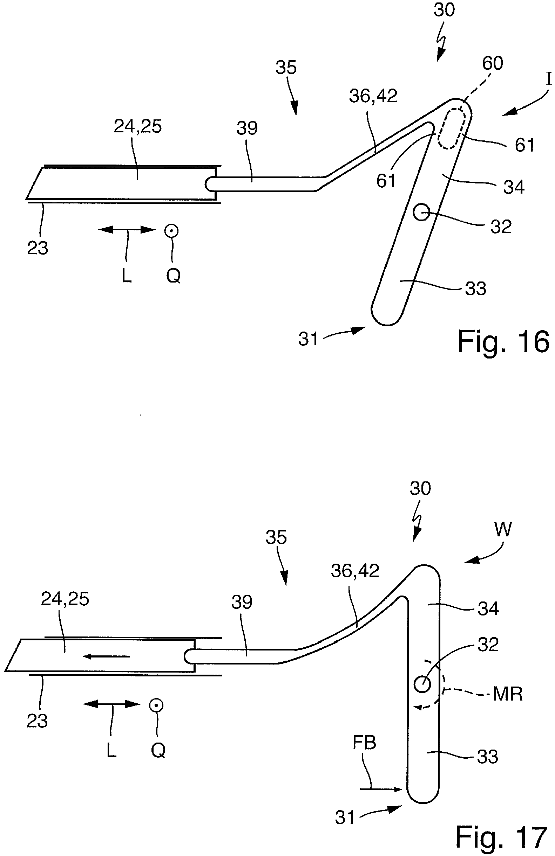

Das Kopplungsglied 36 ist erfindungsgemäß elastisch verformbar ausgebildet. Das Kopplungsglied 36 ist bei den hier beschriebenen bevorzugten Ausführungsbeispielen integral ausgebildet und weist somit keine Naht- oder Fügestelle auf. Es kann gegenüber dem Betätigungselement 31 ein separates Bauteil bilden (Figuren 3-15) oder alternativ integral mit dem Betätigungselement 31 ausgebildet sein. Das Kopplungsglied 36 kann zusätzlich oder alternativ integral mit dem Verbindungsteil 39 ausgebildet sein (Figuren 16 und 17). In Abwandlung zu dem in den Figuren 16 und 17 veranschaulichten Ausführungsbeispiel kann das Kopplungsglied 36 auch lediglich entweder mit dem Betätigungselement 31 oder mit dem Verbindungsteil 39 integral ausgebildet sein und ansonsten so angeordnet bzw. befestigt sein, wie es bei den anderen Ausführungsbeispielen dargestellt bzw. erläutert ist.

Bei den hier beschriebenen bevorzugten Ausführungsbeispielen bildet das Kopplungsglied 36 ein Pleuel 42. Das Pleuel 42 ist dazu eingerichtet, die Schwenkbewegung des zweiten Arms 34 in eine lineare Bewegung in Längsrichtung L des Werkzeugs 24 bzw. des Verbindungsteils 39 zu überführen. Beispielsgemäß ist das erste Ende 37 daher am zweiten Arm 34 angeordnet und mithin um die Schwenkachse S bzw. das Schwenkgelenk 32 schwenkbar gelagert. Das erste Ende 37 des Kopplungsgliedes 36 ist mit Abstand zur Schwenkachse bzw. zum Schwenkgelenk 32 angeordnet. Das zweite Ende 38 des Kopplungsgliedes 36 ist in Längsrichtung L gemeinsam mit dem Werkzeug 24 bzw. dem Verbindungsteil 39 verschiebbar angeordnet. Daher kann das Kopplungsglied 36 auch als Pleuel 42 bezeichnet werden.

Bei dem in den Figuren 3-5 veranschaulichten Ausführungsbeispiel der Betätigungsvorrichtung 30 weist das Kopplungsglied 36 einen ersten Längsschenkel 43 und einen zweiten Längsschenkel 44 auf. Die beiden Längsschenkel 43, 44 sind in Querrichtung Q mit Abstand zueinander angeordnet. Am zweiten Ende 38 sind die beiden Längsschenkel 43, 44 über einen sich in Querrichtung Q erstreckenden Querschenkel 45 miteinander verbunden und beispielsgemäß integral ausgebildet.

Am ersten Ende 37 weist der erste Längsschenkel 43 ein erstes freies Schenkelende 46 und der zweite Längsschenkel 44 ein zweites freies Schenkelende 47 auf. Die freien Schenkelenden 46, 47 sind gegenüber den Längsschenkeln 43, 44 umgebogen oder abgewinkelt und erstrecken sich beim Ausführungsbeispiel im Wesentlichen parallel zum Querschenkel 45, also in Querrichtung Q. Das erste freie Schenkelende 46 und das zweite freie Schenkelende 47 sind beispielsgemäß kürzer als der Querschenkel 45.

Wie es insbesondere in den Figuren 4 und 5 zu erkennen ist, sind die freien Schenkelenden 46, 47 in einer Projektionsebene, die rechtwinklig zur Querrichtung Q ausgerichtet ist, versetzt bzw. mit Abstand zueinander angeordnet. Dies ist beim Ausführungsbeispiel dadurch erreicht, dass der erste Längsschenkel 43 vom Querschenkel 45 bis zum ersten freien Schenkelende 46 eine größere Länge aufweist als der zweite Längsschenkel 44 vom Querschenkel 45 bis zum zweiten freien Schenkelende 47.

Der Querschenkel 45 kann sich durch ein Durchgangsloch in Querrichtung Q durch das Verbindungsteil 39 erstrecken. Alternativ dazu kann der Querschenkel 45 auch in eine oben offene Nut am Verbindungsteil 39 und/oder am Werkzeug 24 eingreifen und optional eingerastet werden, so dass eine lösbare Verbindung zwischen dem Werkzeug 24 und der Kopplungseinrichtung 35 hergestellt ist. Das kann die Montage und/oder den Austausch des Werkzeugs unabhängig von der Kopplungseinrichtung 35 vereinfachen.

Die beiden freien Schenkelenden 46, 47 können jeweils in ein Aufnahmeloch im zweiten Arm 34 hineinragen. Beispielsgemäß ist das Kopplungsglied 36 drehbar bzw. gelenkig sowohl am Verbindungsteil 39, als auch am zweiten Arm 34 gelagert.

In der Ausgangslage I des Betätigungselements 31 nimmt das Kopplungsglied 36 einen ersten Zustand ein. Im ersten Zustand kann das Kopplungsglied elastisch unverformt sein. In der Arbeitslage W des Betätigungselements 31 nimmt das Kopplungsglied 36 einen gegenüber dem ersten Zustand elastisch verformten zweiten Zustand ein.

Im ersten Zustand des Kopplungsgliedes 36 erstrecken sich die beiden Längsschenkel 43, 44 und der Querschenkel 45 in einer gemeinsamen Ebene (Figur 3). Wird das Betätigungselement 31 aus seiner Ausgangslage I heraus geschwenkt werden die beiden Längsschenkel 43, 44 in einer Projektionsebene betrachtet, die sich rechtwinklig zur Querrichtung Q erstreckt, relativ zueinander geneigt. Dies ist darauf zurückzuführen, dass die Lagerstellen der beiden freien Schenkelenden 46, 47 außerhalb der Ausgangslage I nicht mehr gemeinsam mit dem Querschenkel 45 in einer Ebene liegen. Das Neigen der beiden Längsschenkel 43, 44 relativ zueinander außerhalb der Ausgangslage I führt dazu, dass der Querschenkel 45 tordiert wird und dadurch ein Torsionsmoment MT in Umfangsrichtung um den Querschenkel 45 erzeugt wird, das die beiden Längsschenkel 43, 44 dazu drängt, wieder die Position in der gemeinsamen Ebene mit dem Querschenkel 45 einzunehmen. Dadurch wird gleichzeitig ein Rückstellmoment MR auf das Betätigungselement 31 bewirkt, das das Betätigungselement 31 zurück in seine Ausgangslage I drängt.

Beispielsgemäß sind die beiden freien Schenkelenden 46, 46 in der Arbeitslage W des Betätigungselements 31 auf derselben Seite bezüglich einer sich rechtwinkelig zur Längsrichtung L und entlang der Schwenkachse des Betätigungselements 31 erstreckenden Bezugsebene B angeordnet (Figur 4). Insbesondere sind die beiden freien Schenkelenden 46, 46 in der Arbeitslage W des Betätigungselements 31 auf der einen Seite und das zweite Ende 38 des Kopplungsgliedes 36 auf der anderen Seite der Bezugsebene B angeordnet.

Somit bildet das Kopplungsglied 36 nicht nur ein mechanisches Element, über das die Bewegungskopplung zwischen dem Betätigungselement 31 und dem Werkzeug 24 hergestellt wird, sondern gleichzeitig auch eine Rückstelleinrichtung, wodurch das Betätigungselement 31 zurück in seine Ausgangslage I gedrängt wird. Ohne das Aufbringen einer Betätigungskraft FB auf das Betätigungselement 31 nimmt es die Ausgangslage I ein und das Werkzeug 24 und beispielsgemäß das Messer 25 wird in die eingefahrene Stellung E zurückgezogen. Zusätzliche elastisch verformbare Rückstellelemente, insbesondere Schraubenfedern, können entfallen. Dadurch ist der Aufbau der Kopplungseinrichtung 35 sehr einfach. Die Anzahl der benötigten Elemente in der Kopplungseinrichtung 35 bzw. der Betätigungsvorrichtung 30 ist reduziert. Der einfache Aufbau erleichtert auch das Reinigen. Insbesondere durch das Vermeiden von Schraubenfedern, bei denen die Windungen eng aneinander anliegen können, ist das Reinigen bzw. Sterilisieren wesentlich erleichtert.

In den Figuren 3 und 4 ist außerdem eine weitere optionale Ausgestaltungsmöglichkeit der Betätigungsvorrichtung 30 stark schematisiert dargestellt. Das Kopplungsglied 36 erzeugt beispielsgemäß auch eine Kraftkomponente in einer Richtung rechtwinklig zur Längsrichtung L und rechtwinklig zur Querrichtung Q, die als Andrückkraft FA bezeichnet ist. Diese Kraftkomponente entsteht, wenn beim Ausführungsbeispiel gemäß der Figuren 3 und 4 der Querschenkel 45 tordiert wird, also zumindest außerhalb der Ausgangslage I. Diese Andrückkraft FA kann genutzt werden, um einen Rastvorsprung 48 am Werkzeug 24 und/oder am Verbindungsteil 39 in eine zugeordnete Rastaussparung 49 benachbart zum Werkzeugkanal 23 einzudrücken. Es ist auch möglich, dass mehrere Rastvorsprünge 48 und/oder mehrere Rastaussparungen 49 vorhanden sind. Dadurch kann das Werkzeug 24 während der Bewegung in Längsrichtung L in wenigstens einer Stellung einrasten. Einer Bedienperson kann durch das Einrasten eine Rückmeldung über die aktuell erreichte Position des Werkzeugs 24 relativ zum Werkzeugkanal 23 bzw. relativ zu den Branchen 21, 22 gegeben werden. In Figur 4 ist das Einrasten in der ausgefahrenen Stellung A des Werkzeugs 24 veranschaulicht, wobei die Raststellung auch in wenigstens einer beliebigen anderen definierten Stellung des Werkzeugs 24 gegenüber dem Werkzeugkanal 23 vorgegeben sein kann.

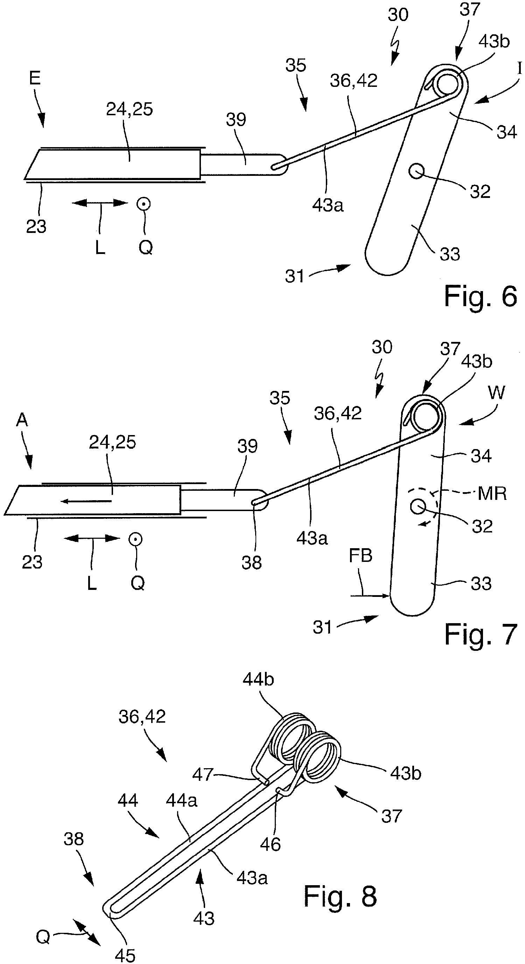

In den Figuren 6-8 ist ein weiteres Ausführungsbeispiel der Betätigungsvorrichtung 30 bzw. eines Kopplungsgliedes 36 veranschaulicht. Abgesehen von der Ausführungsform des Kopplungsgliedes 36, kann die Ausführungsform der Betätigungsvorrichtung 30 der anhand der Figuren 3-5 zuvor beschriebenen Ausführungsform entsprechen. Es wird daher auf die vorstehende Beschreibung verwiesen.

Das abgewandelte Kopplungsglied 36 ist insbesondere in Figur 8 zu erkennen. Analog zum Ausführungsbeispiel gemäß Figur 5 weist das Kopplungsglied 36 eine bügelförmige Gestalt auf mit einem ersten Längsschenkel 43, einem zweiten Längsschenkel 44 und einem Querschenkel 45 am zweiten Ende 38. Das entgegengesetzte erste Ende 37 des Kopplungsgliedes 36 ist anders ausgestaltet als beim Ausführungsbeispiel nach Figur 5. Beide Längsschenkel 43, 44 sind vorzugsweise gleich lang und die sich in Querrichtung Q erstreckenden freien Schenkelenden 46, 47 fluchten vorzugsweise entlang einer gemeinsamen Geraden in Querrichtung Q. Jeder Längsschenkel 43, 44 hat einen sich ausgehend vom Querschenkel 45 geradlinig erstreckenden geraden Schenkelabschnitt 43a, 44a und einen sich daran unmittelbar anschließenden gebogenen Schenkelabschnitt 43b, 44b. Der gebogene Schenkelabschnitt 43b, 44b weist im nicht durch eine Kraft beaufschlagten Ruhezustand einen bogenförmigen und insbesondere kreisbogenförmigen Verlauf auf. Bei dem in Figur 8 dargestellten Ausführungsbeispiel weist der gebogene Schenkelabschnitt 43b, 44b wenigstens eine vollständige Windung und beispielsgemäß mehrere Windungen auf. Der gewundene oder gebogene Schenkelabschnitt 43b stellt eine Verbindung her zwischen dem betreffenden geraden Schenkelabschnitt 43a, 44a und dem jeweiligen Schenkelende 46, 47. Das Kopplungsglied 36 gemäß Figur 8 ist integral ausgebildet und kann beispielsweise durch das Biegen eines Drahtes hergestellt werden. Wie es in Figur 8 zu erkennen ist, sind die freien Schenkelenden 46, 47 näher am Querschenkel 45 angeordnet als die gewundenen oder gebogenen Schenkelabschnitte 43b, 44b.

Analog zu dem Ausführungsbeispiel gemäß der Figuren 3-5 durchsetzt der Querschenkel 45 ein Durchgangsloch im Verbindungsteil 39 oder alternativ im Werkzeug 24. Die freien Schenkelenden 46, 47 greifen jeweils in eine Aussparung oder Vertiefung am zweiten Arm 34 ein.

Wird nun das Betätigungselement 31 durch Aufbringen einer Betätigungskraft FB aus der Ausgangslage I heraus bewegt, ändert sich die Spannung im jeweiligen gewundenen oder gebogenen Schenkelabschnitt 43b, 44b und das Kopplungsglied 36 versucht den Zustand einzunehmen, den es in der Ausgangslage I hat. Auf diese Weise wird ein Rückstellmoment MR um die Schwenkachse bzw. das Schwenkgelenk 32 erzeugt.

Sowohl beim Ausführungsbeispiel gemäß der Figuren 3-5 als auch beim Ausführungsbeispiel gemäß der Figuren 6-8 sind die Längsschenkel 43, 44 bzw. die geraden Schenkelabschnitte 43a, 44a biegesteif gegen eine Biegung quer zu ihrer Erstreckungsrichtung. Dadurch wird eine von extern auf das Werkzeug 24 einwirkende Kraft sehr gut über das Kopplungsglied 36 am Betätigungselement 31 abgestützt.

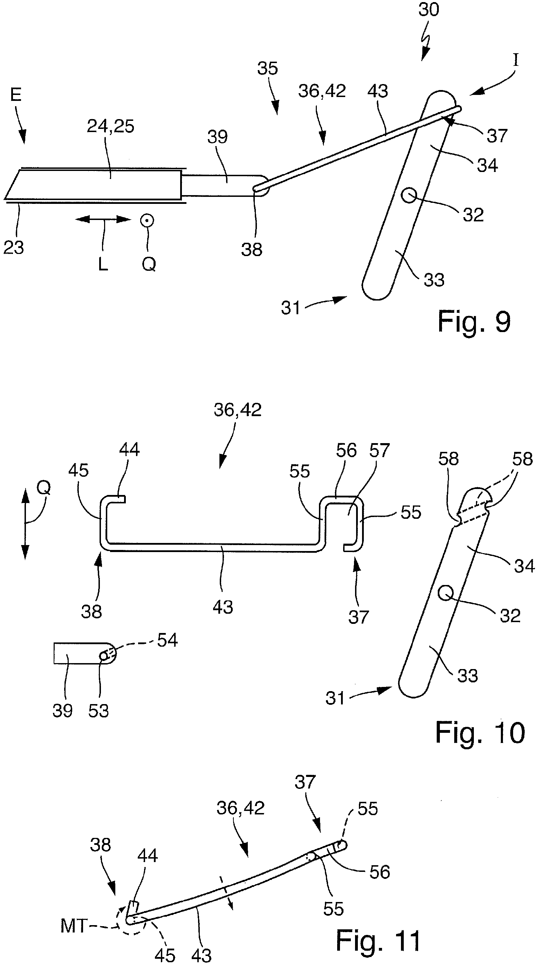

Aus den Figuren 9-11 geht eine weitere Ausführungsform der Betätigungsvorrichtung 30 hervor, die ähnlich ist zu der Ausführungsform gemäß der Figuren 3-5, so dass auf die Beschreibung zu diesen Figuren verwiesen werden kann. Der wesentliche Unterschied besteht bei diesem Ausführungsbeispiel darin, dass lediglich ein Längsschenkel 43 vorhanden ist, der den Querschenkel 45 am zweiten Ende 38 mit dem ersten Ende 37 verbindet. Der zweite Längsschenkel 44 erstreckt sich ausgehend vom Querschenkel 45 lediglich im Bereich des zweiten Endes 38 und dient dort zur gegenüber dem Querschenkel 45 drehfesten Anordnung des Kopplungsgliedes 36 am Werkzeug 24 und beispielsgemäß am Verbindungsteil 39. Dazu kann der kurze zweite Längsschenkel 44 beispielsweise in einer sich an ein Durchgangsloch 53 für den Querschenkel 45 anschließenden Nut 54 angeordnet sein. Der Querschenkel 45 bleibt um eine Erstreckungsrichtung tordierbar, wenn sich der erste Längsschenkel 43 durch das Betätigen des Betätigungselements 31 gegenüber dem drehfest angeordneten zweiten Längsschenkel 44 bewegt bzw. schwenkt.

Das erste Ende 37 des Kopplungsgliedes 36 ist am Betätigungselement 31 fixiert. Zum Beispiel kann das Kopplungsglied 36 am ersten Ende 37 mehrfach gebogen sein, so dass zwei sich in Querrichtung Q erstreckende Halteschenkel 55 gebildet sind, die über einen Verbindungsschenkel 56 miteinander verbunden sind. Im Bereich zwischen den beiden Halteschenkeln 55 ist ein Zwischenraum 57 begrenzt, in den der zweite Arm 34 eingreifen kann. Die Halteschenkel 55 und der Verbindungsschenkel 56 umgreifen sozusagen den zweiten Arm 34 des Betätigungselements 31. Zur Fixierung kann im zweiten Arm 34 eine Haltenut 58 vorhanden sein, in der zumindest die Halteschenkel 55 und/oder der Verbindungsschenkel 56 angeordnet wird.

In Figur 11 ist stark schematisiert die Kraft- und Momentenbeanspruchung des Kopplungsgliedes 36 veranschaulicht, wenn das Betätigungselement 31 aus seiner Ausgangslage I ausgelenkt wird. Durch das Schwenken der beiden Längsschenkel 43, 44 zueinander um den Querschenkel 45 entsteht am Querschenkel 45 ein Torsionsmoment MT. Zusätzlich kann der erste Längsschenkel 43 aus seiner geradlinigen Erstreckung heraus gebogen werden. Durch diese Kräfte und Momente entsteht ein Rückstellmoment MR um die Schwenkachse S bzw. das Schwenkgelenk 32, das das Betätigungselement 31 in seine Ausgangslage I zurück drängt.

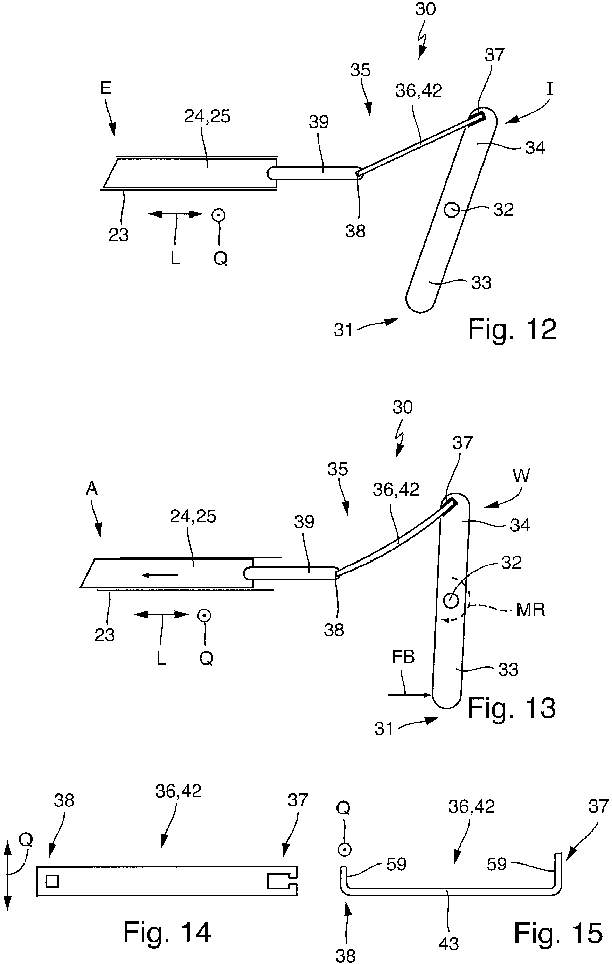

Bei dem in den Figuren 12 und 13 veranschaulichten Ausführungsbeispiel der Betätigungsvorrichtung 30 entspricht der Aufbau den zuvor beschriebenen Ausführungsbeispielen abgesehen von der Ausgestaltung des Kopplungsgliedes 36. Das Kopplungsglied 36 ist bei dieser Ausführungsform so ausgestaltet, dass es aus seiner ursprünglichen Erstreckung in der Ausgangslage I des Betätigungselements 31 gebogen wird und dadurch das Rückstellmoment MR bewirkt.

In den Figuren 14 und 15 sind Ausführungsbeispiele für das Kopplungsglied 36 veranschaulicht. Das Kopplungsglied 36 kann die Gestalt einer Blattfeder aufweisen, wie es schematisch in Figur 14 veranschaulicht ist. Das Kopplungsglied 36 wird am ersten Ende 37 und am zweiten Arm 34 und am zweiten Ende 38 am Werkzeug 24 oder am Verbindungsteil 39 in der Ausrichtung fixiert, die es in der Ausgangslage I einnimmt. Dadurch wird beim Schwenken des Betätigungselements 31 eine Biegung des Kopplungsgliedes 36 erzeugt. Das Kopplungsglied 36 versucht wieder seine Ursprungslage einzunehmen und erzeugt auf diese Weise das Rückstellmoment MR.

Zur Fixierung des Kopplungsgliedes 36 kann dieses am ersten Ende 37 oder am zweiten Ende 38 wenigstens eine Durchbrechung und/oder wenigstens eine nach außen offene Aussparung aufweisen. Die die Aussparung begrenzenden Teile können in Nuten oder Ausnehmungen angeordnet werden, beispielsweise am zweiten Arm 34. Mittels der Durchbrechung kann das Kopplungsglied 36 beispielsweise am zweiten Ende 38 mit dem Material des Verbindungsteils 39 umspritzt werden. Die Verbindung am ersten Ende 37 und am zweiten Ende 38 kann formschlüssig und/oder kraftschlüssig und/oder stoffschlüssig und/oder eine Haftverbindung sein. Ein Umspritzen des Kopplungsgliedes 36 ist vorzugsweise zumindest an einem der beiden Enden 37 oder 38 oder alternativ auch an beiden Enden 37 und 38 möglich.

Anstelle eines blattfederähnlichen Kopplungsgliedes 36, wie es in Figur 14 veranschaulicht ist, kann das Kopplungsglied 36 auch durch ein Drahtelement oder Drahtbügelelement gebildet sein (Figur 15). Es besteht im Wesentlichen aus einem Längsschenkel 43, der sich zwischen dem ersten Ende 37 und dem zweiten Ende 38 erstreckt und der sowohl am ersten Ende 37, als auch am zweiten Ende 38 in einem rechtwinklig zum Längsschenkel 43 verlaufenden Endschenkel 59 übergeht. Die beiden Endschenkel 59 erstrecken sich vorzugsweise parallel zueinander und insbesondere in einer Ebene, die rechtwinklig zur Querrichtung aufgespannt ist. Die beiden Endschenkel 59 können in entsprechende Löcher oder Aussparungen am zweiten Arm 34 bzw. am Werkzeug 24 oder Verbindungsteil 39 eingesteckt und dort gegebenenfalls fixiert werden, beispielsweise durch eine Haftverbindung. Beim Auslenken des Betätigungselements 31 aus seiner Ausgangslage I wird der Längsschenkel 43 aus seiner ursprünglichen Erstreckung heraus gebogen, da die Endschenkel 59 an den beiden Enden 37, 38 in ihrer Lage relativ zum zweiten Arm 34 einerseits und relativ zum Werkzeug 24 bzw. zum Verbindungsteil 39 andererseits fixiert sind.

Ein weiteres Ausführungsbeispiel der Betätigungsvorrichtung 30 ist in den Figuren 16 und 17 veranschaulicht. Während das Kopplungsglied 36 bei den vorstehend beschriebenen Ausführungsbeispielen ein separates Bauteil war, ist das Kopplungsglied 36 beim Ausführungsbeispiel gemäß der Figuren 16 und 17 integral mit dem Betätigungselement 31 und/oder integral mit dem Verbindungsteil 39 ausgebildet. In der dargestellten Ausführungsform sind das Betätigungselement 31, das Kopplungsglied 36 und das Verbindungsteil 39 ein einziges integrales Bauteil, das beispielsweise aus Kunststoff besteht und insbesondere als Spritzgussteil ausgebildet sein kann. Wenn das Kopplungsglied 36 nur mit dem Betätigungselement 31 oder nur mit dem Verbindungsteil 39 integral ausgebildet ist, kann es am jeweils anderen Ende 37 bzw. 38 kraftschlüssig und/oder formschlüssig und/oder stoffschlüssig und/oder durch Haftvermittlung mit dem Betätigungselement 31 bzw. mit dem Verbindungsteil 39 verbunden sein.

Das Kopplungsglied 36 bildet analog zu den Ausführungsbeispielen gemäß der Figuren 12-15 eine Biegefeder und kann somit die Form einer Blattfeder ähnlich wie die Ausführungsform gemäß Figur 14 oder eine stegförmige Gestalt ähnlich wie der gerade Längsschenkel 43 gemäß Figur 15 aufweisen. Die Funktionsweise entspricht der des Ausführungsbeispiels gemäß der Figuren 12 und 13.

In Figur 16 ist außerdem eine weitere optionale Möglichkeit veranschaulicht. Zusätzlich dazu, dass das Rückstellmoment MR durch eine Verformung des Kopplungsgliedes 36 bewirkt wird, kann das Rückstellmoment MR zumindest teilweise auch durch eine Verformung des zweiten Arms 34 des Betätigungselements 31 erzeugt werden. Um diese Verformung zu ermöglichen, kann der zweite Arm 34 eine entsprechende verformbare Konfiguration aufweisen. In Figur 16 ist dies beispielhaft durch eine Durchbrechung 60 veranschaulicht, die den zweiten Arm durchsetzt. Benachbart zu der Durchbrechung 60 weist der zweite Arm 34 stegförmige Abschnitte 61 auf, die im Querschnitt ausreichend gering dimensioniert sind. Dadurch ist ein Biegen oder Verformen der stegförmigen Abschnitte 61 und/oder eine Bewegung der stegförmigen Abschnitte aufeinander zu ermöglicht. Ist dieser Bereich des zweiten Arms 34 verformt (außerhalb der Ausgangslage I), dann tendiert der zweite Arm 34 dazu, seine unverformte Ausgangsgestalt anzunehmen und erzeugt dadurch zumindest eine Komponente des Rückstellmoments MR.

Bei einer weiteren Abwandlung der Betätigungsvorrichtung 30 wäre es auch möglich, das Kopplungsglied 36 bei den auftretenden Kräften unverformbar steif auszugestalten und lediglich zumindest einen Teil des zweiten Arms 34 verformbar auszuführen, um das Rückstellmoment MR zu erzeugen.

Bei sämtlichen bügelförmigen Ausgestaltungen des Kopplungsgliedes 36, insbesondere den Ausgestaltungen gemäß der Figuren 3-11 und 15, kann das Kopplungsglied 36 durch ein Drahtbiegeteil gebildet sein. Der Draht kann einen kreisförmigen Querschnitt aufweisen. Vorzugsweise besteht der Draht aus einer metallischen Legierung.

Die Verformbarkeit, zumindest eines Teils des zweiten Arms 34 und/oder die im Zusammenhang mit den Figuren 3 und 4 erläuterten Rastmittel 48, 49, können bei sämtlichen Ausführungsbeispielen vorhanden sein.

Bei allen Ausführungen kann eine lösbare Verbindung zwischen dem Werkzeug 24 und der Kopplungseinrichtung 35 mittelbar über das Verbindungsteil 39 oder auch unmittelbar hergestellt werden, beispielsweise eine kraftschlüssige und/oder formschlüssige Verbindung, vorzugsweise eine Rastverbindung.

Die Erfindung betrifft eine Betätigungsvorrichtung 30 zur Betätigung eines Werkzeugs 24 eines chirurgischen Instruments 20. Die Betätigungsvorrichtung 30 hat ein Betätigungselement 31, das zwischen einer Ausgangslage I und einer Arbeitslage W schwenkbar gelagert ist. Mittels einer Kopplungseinrichtung 35, die ein Kopplungsglied 36 aufweist oder durch das Kopplungsglied 36 gebildet ist, ist das Betätigungselement 31 mit dem Werkzeug 24 bewegungsgekoppelt. Das Kopplungsglied 36 ist elastisch verformbar und bewirkt bei einer Auslenkung des Betätigungselements 31 aus der Ausgangslage I durch die elastische Verformung ein Rückstellmoment MR, das das Betätigungselement 31 zurück in die Ausgangslage I drängt. Zusätzliche federelastische Rückstellelemente können dadurch entfallen.

### Bezugszeichenliste:

- 20: Instrument
- 21: Branche
- 21a: Gewebekontaktfläche
- 22: Branche
- 22a: Gewebekontaktfläche
- 23: Werkzeugkanal
- 24: Werkzeug
- 25: Messer
- 26: Schaft
- 27: Bedieneinheit

- 30: Betätigungsvorrichtung
- 31: Betätigungselement
- 32: Schwenkgelenk
- 33: erster Arm
- 34: zweiter Arm
- 35: Kopplungseinrichtung
- 36: Kopplungsglied
- 37: erstes Ende des Kopplungsglieds
- 38: zweites Ende des Kopplungsglieds
- 39: Verbindungsteil

- 42: Pleuel
- 43: erster Längsschenkel
- 43a: gerader Schenkelabschnitt des ersten Längsschenkels
- 43b: gebogener Schenkelabschnitt des ersten Längsschenkels
- 44: zweiter Längsschenkel
- 44a: gerader Schenkelabschnitt des zweiten Längsschenkels
- 44b: gebogener Schenkelabschnitt des zweiten Längsschenkels
- 45: Querschenkel
- 46: erstes freies Schenkelende
- 47: zweites freies Schenkelende
- 48: Rastvorsprung
- 49: Rastaussparung

- 53: Durchgangsloch
- 54: Nut
- 55: Halteschenkel
- 56: Verbindungsschenkel
- 57: Zwischenraum
- 58: Haltenut
- 59: Endschenkel
- 60: Durchbrechung
- 61: stegförmige Abschnitt

- A: ausgefahrene Stellung des Werkzeugs
- B: Bezugsebene
- E: eingefahrene Stellung des Werkzeugs
- FA: Andrückkraft
- FB: Betätigungskraft
- I: Ausgangslage
- L: Längsrichtung
- MR: Rückstellmoment
- MT: Torsionsmoment
- Q: Querrichtung
- W: Arbeitslage

## Patentansprüche

1. Chirurgisches Instrument (20) aufweisend:
- ein Werkzeug (24), das in einer Längsrichtung (L) geführt bewegbar gelagert ist,
- ein Betätigungselement (31), das schwenkbar gelagert ist, um zwischen einer Ausgangslage (I) und einer Arbeitslage (W) geschwenkt zu werden,
- eine Kopplungseinrichtung (35), die eine Bewegungskopplung zwischen dem Betätigungselement (31) und dem Werkzeug (24) herstellt, wobei die Kopplungseinrichtung (35) ein Kopplungsglied (36) aufweist, das elastisch verformbar ist und in der Arbeitslage (W) des Betätigungselements (31) derart elastisch verformt ist, dass es ein Rückstellmoment (MR) in Richtung der Ausgangslage (I) auf das das Betätigungselement (31) bewirkt.

2. Chirurgisches Instrument nach Anspruch 1, wobei das Kopplungsglied (36) ein Pleuel (42) bildet.

3. Chirurgisches Instrument nach Anspruch 1 oder 2, wobei das Kopplungsglied (36) mit einem ersten Ende (37) unmittelbar am Betätigungselement (31) angeordnet ist und/oder wobei das Kopplungsglied (36) mit einem zweiten Ende (38) unmittelbar mit dem Werkzeug (24) oder einem unbeweglich am Werkzeug (24) angeordneten Verbindungsteil (39) angeordnet ist.

4. Chirurgisches Instrument nach einem der vorhergehenden Ansprüche, wobei das Kopplungsglied (36) wenigstens einen Längsschenkel (43, 44) und einen sich daran anschließenden Querschenkel (45) aufweist, der sich in einer Querrichtung (Q) rechtwinkelig zur Längsrichtung (L) erstreckt.

5. Chirurgisches Instrument nach einem der vorhergehenden Ansprüche, wobei das Kopplungsglied (36) zwei mit Abstand zueinander angeordnete Längsschenkel (43, 44) aufweist, die über einen Querschenkel (45) miteinander verbunden sind.

6. Chirurgisches Instrument nach Anspruch 5, wobei jeder Längsschenkel (43, 44) ein freies Schenkelende (46, 47) aufweist, die versetzt zueinander angeordnet sind.

7. Chirurgisches Instrument nach Anspruch 5 oder 6, wobei die Längsschenkel (43, 44) unterschiedlich lang sind.

8. Chirurgisches Instrument nach einem der Ansprüche 5 bis 7, wobei die Längsschenkel (43, 44) und der Querschenkel (45) in der Ausgangslage (I) des Betätigungselements (31) in einer gemeinsamen Ebene angeordnet sind.

9. Chirurgisches Instrument nach einem der Ansprüche 5 bis 8, wobei die Längsschenkel (43, 44) in der Arbeitslage (W) des Betätigungselements (31) einen Winkel ungleich null einschließen.

10. Chirurgisches Instrument nach einem der Ansprüche 4 bis 9, wobei der wenigstens eine Längsschenkel (43, 44) in der Arbeitslage (W) des Betätigungselements (31) gegenüber der Ausgangslage (I) elastisch unverformt ist.

11. Chirurgisches Instrument nach einem der Ansprüche 4 bis 10, wobei der Querschenkel (45) in der Arbeitslage (W) des Betätigungselements (31) um seine Erstreckungsrichtung tordiert ist.

12. Chirurgisches Instrument nach einem der vorhergehenden Ansprüche, wobei sich das Kopplungsglied (36) in der Ausgangslage (I) des Betätigungselements (31) geradlinig zwischen seinen beiden Enden (37, 38) erstreckt.

13. Chirurgisches Instrument nach einem der vorhergehenden Ansprüche, wobei sich das Kopplungsglied (36) in der Arbeitslage (W) des Betätigungselements (31) gekrümmt zwischen seinen beiden Enden (37, 38) erstreckt.

14. Chirurgisches Instrument nach einem der vorhergehenden Ansprüche, wobei das in der Arbeitslage (W) des Betätigungselements (31) elastisch verformte Kopplungsglied (36) eine Kraft (FA) rechtwinkelig zur Längsrichtung (L) auf das Werkzeug (24) oder ein mit dem Werkzeug (24) verbundenes Verbindungsteil (39) ausübt.
